# EUROPÄISCHE PATENTSCHRIFT

(11) **EP 1 818 083 B1**
(45) Veröffentlichungstag und Bekanntmachung des Hinweises auf die Patenterteilung: **25.02.2009**
(21) Anmeldenummer: 07001568.0
(22) Anmeldetag: 25.01.2007
(51) Int. Cl.: A63B 24/00, A61B 5/107

(54) **Messvorrichtung**
Measuring device
Dispositif de mesure

(30) Priorität: 03.02.2006 DE 102006005409
(43) Veröffentlichungstag der Anmeldung: 15.08.2007
(73) Patentinhaber: milon industries GmbH, 86494 Emersacker (DE)
(72) Erfinder: Dieter Miehlich, 86494 Emersacker (DE)
(74) Vertreter: Vossius, Corinna

(56) Entgegenhaltungen:
- WO-A2-20/05074370
- DE-A1- 10 308 267
- DE-A1- 19 509 268
- FR-A1- 2 770 992
- US-A- 5 421 798

## Beschreibung

Die Erfindung betrifft eine Messvorrichtung zum Ermitteln von körperbezogenen Parameterwerten zu vermessender Testpersonen, mit denen Fitnessgeräte auf die jeweilige Person eingestellt werden können, gemäß dem Oberbegriff des Anspruchs 1.

An sich bekannt sind Fitness- bzw. Trainingsgeräte, die sich automatisiert immer wieder auf einmal voreingestellte personenbezogene Parameter einstellen lassen. Dazu werden die personenbezogenen Einstellungen beispielsweise auf einer Chipkarte gespeichert, die bei Trainingsbeginn an dem Gerät eingelesen wird, so dass sich das Gerät bei jedem Training auf die jeweilige Testperson einstellen kann.

Bei der Erst- bzw. Voreinstellung muss das jeweilige Fitnessgerät jedoch per Hand oder jedenfalls durch menschliche Bedienung auf die Körpergeometrie des Probanden eingestellt werden. Diese Ersteinstellung muss kostenintensiv von geschultem Personal, wie z.B. einem Trainer oder Therapeut vorgenommen werden, wobei die Daten der Ersteinstellung dann auf der Chipkarte oder einem anderen transportablen oder stationären Datenträger gespeichert werden. Insbesondere in Fitnesseinrichtungen, die ein Trainierender ohne Betreuer aufsucht, wie beispielsweise hoteleigenen Fitnessanlagen ist dies jedoch nicht möglich, wodurch es immer wieder zu Fehleinstellungen, Fehlbedienungen der Fitnessgeräte durch die Trainierenden und damit bestenfalls zu suboptimalen Trainingsergebnissen, schlimmstenfalls aber auch zu körperlichen Schäden der Trainierenden kommt.

Eine Möglichkeit, die manuelle Fitnessgeräte-Voreinstellung durch geschultes Personal zumindest zu erleichtern, ist der US-Patentschrift US 5,454,773 zu entnehmen. Darin ist eine Muskeltrainings- und Rehabilitationsvorrichtung gezeigt, die einen Stuhl für den Trainierenden und einen von ihm zu Trainingszwecken zu betätigenden Aktuator aufweist, wobei der Aufbau der dortigen Vorrichtung so hohe Flexibilität aufweist, dass die Drehachse des Aktuators mit derjenigen des zu bewegenden Gelenks abgestimmt werden kann.

So ist dort ein Aufbau der Vorrichtung gezeigt, mit dem das rechte Knie des Trainierenden einer Beugung-Streckung unterzogen wird, wobei die Drehachse des drehbaren Aktuators horizontal mit dem Gelenk des rechten Knies abgestimmt wird. Zu diesem Zweck ist dort ein Patientenpositioniersystem vorgesehen, bei dem über ein Bedienpanel die Position des Patienten bezüglich des Aktuators eingestellt werden kann, wozu der Stuhl und der Aktuator in verschiedenen Richtungen beweglich zueinander sind. Bei der Ersteinstellung der dortigen Vorrichtung muss die geeignete Position per Hand eingegeben werden, bei den darauf folgenden Trainingseinheiten kann die Positionierung des Stuhls zu dem Aktuator jedoch gemäß vorher in dem Speicher eines Rechners abgespeicherten Daten erfolgen. Ferner umfasst die Vorrichtung eine Lastzelle, die mit dem Aktuator verbunden ist, um die von dem Patienten aufgebrachte Kraft zu messen.

Ein weiteres Fitnessgerät, bei dem Einstellungen entsprechend der Abmaße der trainierenden Person vorgenommen werden können und anschließend wieder verwendbar abgespeichert werden können, ist der Offenlegungsschrift DE 101 33 572 A1 zu entnehmen. Jedoch ist auch hier die Ersteinstellung manuell vorzunehmen und erfordert geschultes Personal, wenn gute Trainingserfolge erzielt werden sollen.

Ferner zeigt die US-5,020,795 ein Fitness- und Rehabilitationsgerät mit einem stuhlförmigen Rahmen, an dem ein Aktuator über einen Hebelarm angebracht ist, der vom Trainierenden mit seinem Unterschenkel betätigt werden kann. Auf den Aktuator ist dabei eine Gegenkraft computergesteuert aufbringbar, wobei die aufgebrachte Gegenkraft über den Rechner aufgezeichnet wird und wobei Parameter wie beispielsweise die vom Benutzer aufgebrachte Kraft während der Bewegung des Aktuators, die Distanz, über die diese Kraft ausgeübt wird, die Art und Dauer der Anwendung des Fitnessgeräts etc. gemessen und registriert werden. Dabei ist jedoch die Länge des Hebelarms per Hand auf den jeweiligen Benutzer einzustellen, so dass die gewonnenen Daten abhängig von der fachmännischen Einstellung der Länge des Hebelarms sind.

Auch in weiteren Schriften sind Kraftmessvorrichtungen gezeigt, die entweder für sich oder als in ein Trainingsgerät integrierte Messstation nutzbar sind, um die Kraft von zu trainierenden Muskelpartien von Testpersonen zu erfassen. Dabei sind jeweils Stuhl- oder Liegesitzanordnungen mit entsprechenden Aktuatoren versehen, über die die in dem Sitz aufgenommene Testperson die zu messende Kraft gegen eine vom Gerät aufgebrachte Gegenkraft aufbringen kann. Ferner sind diese Kraftmessgeräte auf die Körpergeometrie der jeweiligen Testperson einstellbar, so dass sichergestellt ist, dass die Kraft mit der gewünschten Muskelpartie auf den Aktuator aufgebracht wird.

So zeigt z.B. das deutsche Gebrauchsmuster DE 20 2004 002 975 U1 einen Messstuhl zum Messen und Dokumentieren von Leistungen verschiedener Muskelgruppen, bei dem ein Arm-/ Beinpolster gegenüber einem Brust-/Rückenpolster vertikal einstell- und fixierbar ist, um unterschiedliche Armlängen oder Beindicken der Testpersonen ausgleichen zu können.

Weiterhin zeigt die DE 2952983 T ein Gerät zur statischen oder dynamischen Messung der Muskelkraft mit einem auf einem Gestell gelagerten Sitz und einem an dem Gestell befestigten Kraftaufnehmer, der gegenüber dem Sitz verschiebbar ist und über einen Motor in eine gewünschte Position, also in eine für eine Testperson passende Position gebracht werden kann. In dieser Position erfolgt dann die Messung einer von der Testperson auf einen mit dem Kraftaufnehmer verbundenen Aktuator aufgebrachten Kraft.

Ferner zeigt die Offenlegungsschrift DE 195 09 268 A1 schon eine Vorrichtung zur Vermessung und zum Training von Kräften und/oder Beweglichkeiten mit einem Stuhl, in dem der Proband Platz nehmen kann, und Angriffsmitteln, an denen die Testperson bzw. der Proband mit verschiedenen Extremitäten bzw. Muskelgruppen angreifen kann, welche mit Kraftaufnehmern bzw. -aufnahmeplatten verbunden sind, mit denen die vom Proband aufgebrachte Kraft gemessen werden kann.

Dort sind auch schon Positionierungsmittel vorgesehen, mit denen die Relativposition des Probanden gegenüber den Kraftaufnahmeplatten eingestellt werden kann. Für die Positionierungsmittel sind Antriebe vorgesehen, welche über eine zentrale Datenverarbeitungsanlage gesteuert werden können, wobei die Daten des bzw. der Probanden in der zentralen Datenverarbeitungsanlage abgelegt sein können, so dass die Einstellung der jeweiligen Position Proband-Kraftaufnehmer automatisch erfolgen kann, sofern denn die personenbezogenen Körpergeometriedaten schon einmal erfasst worden sind.

Zum Gewinnen der abzulegenden Daten aus einer manuellen Ersteinstellung sind dort auch schon Positionsmessmittel vorgesehen, mit denen die einmal eingestellte Relativposition von einem Körperteil und einem Angriffsmittel erfasst werden kann, um diese in der zentralen Datenhaltungsanlage abzuspeichern. Die Ersteinstellung bzw. Eichung der Vermessungs- und Trainingsvorrichtung auf die Körpergeometrie der jeweiligen Testperson muss aber auch dort durch den Sporttherapeuten oder die als Leistungssportler evtl. selbst entsprechend geschulte Testperson vorgenommen werden.

Gegenüber dieser Geräteklasse besteht jedoch der Bedarf, nicht nur Kraft, Leistung, Beweglichkeit und Ausdauer von Probanden zu erfassen, um die jeweiligen Fitnessgeräte auf das Trainingsprogramm des Probanden einzustellen bzw. die entsprechenden Gegengewichte einzustellen, sondern ebenfalls die Ersteinstellung bzw. Eichung der Fitnessgeräte auf die Körpergeometrie der jeweiligen Testperson zu automatisieren, um so einer Fehleinstellung und damit einem suboptimalen Trainingserfolg, aber auch körperlichen Schädigung des Probanden bzw. der Testperson vorzubeugen, die von Zerrungen bis zu Bänderrissen oder Brüchen reichen können, wie sie beispielsweise durch mit den falschen Muskelpartien oder unter falschem Winkel oder Abstand aufgebrachte Kräfte und durch übermäßige Streckungen oder Stauchungen von Körperpartien entstehen.

Zur Erfassung von Körpergeometriemerkmalen ist aus der US-Patentschrift 4,603,486 eine Messkabine bekannt, bei der die Testperson in sitzender oder stehender Position vermessen werden kann. Dazu sind Berührungssensoren vorgesehen, die sicherstellen, dass die Testperson die Messposition korrekt einhält. In dieser dadurch genormten Messposition können dann verschiedene Schieber an den Körper der Testperson heran geschoben werden, woraufhin sich die Abmaße der Testperson ablesen lassen. Mit dieser Vermessungskabine können somit die Abmaße einer Testperson, wie beispielsweise Körpergröße, Schulterbreite, Kniehöhe, Griffweite etc. in einer genormten Messumgebung erfasst werden. Die erfassten Daten können dann personenbezogen in ein Datenerfassungssystem eingelesen werden.

Damit gelingt es zwar, die Abmaße einer Person bzw. den von ihr in einer bestimmten Körperhaltung benötigten Raum zu erfassen, wie dies beispielsweise zur Auslegung von Pilotenkabinen von Kampfflugzeugen nötig ist. Zur Durchführung der einzelnen Messungen ist jedoch Bedienpersonal nötig, welches die einzelnen Messschieber an den Körper der Person für die jeweilige Messung anlegt.

Darüberhinaus sind die durch die vorgenommenen Abmaß-Messungen gewonnenen Daten für die Einstellung von Fitnessgeräten bzw. Rehabilitationsgeräten nur von beschränkter Aussagekraft, da es hier nicht in erster Linie auf die Körperabmaße einer Testperson ankommt, sondern darauf, die Fitnessgeräte so einzustellen, dass die an dem jeweiligen Gerät auszuführende Übung in der dafür optimalen Haltung durchgeführt werden, d.h. dass beispielsweise Gewichte mit in einem bestimmten Winkelbereich abgewinkelten Arm oder Bein gehoben werden oder dass die Sitzhöhe so eingestellt ist, dass der Brustkorb eines Trainierenden auf ein Brustpolster eines Angriffsorgans drückt und nicht der Bauch.

Ferner ist der US 4,603,486 schon zu entnehmen, dass die Griff- und Trittweite der sitzenden Testperson erfasst wird, indem die über die Berührungssensoren fixierte Testperson eine geführte Bewegung - entweder eine Armstreckung mit einem ergriffenen, entlang einer Bewegungsbahn geführten Messstab oder eine Beinstreckung auf einem entlang einer Bewegungsbahn geführten Pedal - ausgeführt wird und der Endwert bei Erreichen der vollständig durchgestreckten Gliedmaße erfasst wird. Auch bei diesen Messungen wird jedoch lediglich ein Körperabmaß erfasst, nämlich die Länge von der Armspitze des ausgestreckten Arms bis zur Rückenlehne bzw. die Beinlänge von der Fußsohle bis zu einem Messpunkt an dem Sitz der Testperson und nicht ein für einen bestimmten Bewegungsablauf an einem Fitnessgerät wichtiger Parameter.

Bekannte Verfahren zum Ermitteln von für den Bewegungsablauf einer Person entscheidenden Körpergeometrie-Parametern sind dagegen teuer, aufwendig und daher zum Einsatz für eine Messvorrichtung zur Ermittlung von Voreinstellparametern von Fitnessgeräten ungeeignet: Zum Einen existieren sog. Körperscanner, bei denen Fotografien des Körpers der Testperson nach biometrischen Merkmalen in entsprechenden Bildbearbeitungsverfahren erfasst werden. Außerdem ist es beispielsweise zur Erstellung von digitalen Filmcharakteren bekannt, den Körper eines Schauspielers mit verschiedenen Bewegungssensoren zu versehen und die Bewegungsabläufe des Schauspielers zu erfassen.

Weiterhin zeigt die US-Schrift US 6,390,951 B1 schon mehrere Fitnessgeräte, die jeweils eine Messvorrichtung aufweisen, die zu einer Ersteinstellung von Fitnessgeräten auf die Physis der trainierenden Person dient, wobei die Drehpunkte an den als Angriffsorgan dienenden Gerätehebeln in Fluchtung mit einem beim Training auf dem Fitnessgerät bewegten Körpergelenk der trainierenden Person gebracht werden soll.

Dazu ist dort eine Lichtquelle vorgesehen, ein Stellmotor, der an einer geeigneten Stelle des Fitnessgeräts angreift, um beispielsweise die Höhe einer Plattform einzustellen, auf der die trainierende Person steht, und eine Betätigungseinrichtung für den Stellmotor. Die Lichtquelle sendet dabei einen Lichtstrahl in koaxialer Richtung zum Drehpunkt des jeweiligen Gerätehebels in Richtung zum Körper der trainierenden Person. Die trainierenden Person muss dann den Auftreffpunkt auf ihren Körper beobachtet und den Stellmotor über die Betätigungseinrichtung so betätigen, dass der Lichtstrahl auf dem Drehpunkt eines Gelenks auftrifft, mit anderen Worten, dass der Gelenkdrehpunkt mit der Drehachse des Angriffsorgans fluchtet.

Abgesehen davon, dass die vom Trainierenden vorzunehmende Ersteinstellung des Fitnessgeräts mit Beobachten des Lichtsstrahls und entsprechendem Betätigen des Stellmotors ohne Einweisung schwierig und relativ fehleranfällig ist, führt die Messung aber immer zu einem nur ungenauen Ergebnis. Denn der Auftreffpunkt des Lichtstrahls wird mit dem Drehpunkt des Gelenks gleichgesetzt. Ob dies tatsächlich der Fall ist kann der Trainierende aber gar nicht mit Sicherheit feststellen. Ferner wird zwar versucht, einen Gelenkdrehpunkt des Trainierenden und des Fitnessgeräts auszufluchten. Weitergehende Erkenntnisse über die Physis des Trainierenden können hier aber nicht gewonnen werden.

Hiervon ausgehend ist es Aufgabe der vorliegenden Erfindung, eine kostengünstig herzustellende und unkompliziert zu bedienende Vorrichtung zu schaffen, mit der ein personenbezogener Wert zumindest eines, sich aus der Gelenkgeometrie der jeweiligen Person ergebenden Einstellparameters von Fitnessgeräten automatisiert ermittelt werden kann.

Diese Aufgabe wird mit den Merkmalen des Anspruchs 1 gelöst.

Es wurde erkannt, dass es für die Einstellung von Fitnessgeräten auf die Körpergeometriemerkmale verschiedener Personen nicht so sehr auf die Körper-Abmaße der jeweiligen Person ankommt als auf die genaue Kenntnis der Lage der Gelenkdrehpunkte, da die vom menschlichen Körper ausführbaren Bewegungen stets Drehbewegungen um ein oder mehrere Gelenke sind. Es kommt für die Einstellung von Fitnessgeräten auf die Körpergeometrie somit in besonderem Maße darauf an, die durch das dem Übenden zur Verfügung gestellte Übungsorgan und dessen Verbindung mit einer Gegenkrafterzeugungseinrichtung definierte Zwangsbewegung, die der Übende an dem jeweiligen Fitnessgerät ausführt, auf die Gelenkdrehachsen des Übenden einzustellen.

Erfindungsgemäß wird daher eine Messvorrichtung vorgeschlagen, mit der eine Höhe zumindest eines Drehpunkts zumindest eines Gelenks unterschiedlicher Personen aus einer standardisierten Körperbewegung der jeweiligen Person ermittelt wird, wobei die Ermittlung der Höhe des Drehpunkts automatisiert erfolgt, d.h. ohne dass Maß genommen werden muss, oder bestimmte Voreinstellungen des Geräts vorzunehmen sind. Dabei ist die standardisierte Körperbewegung ausschließlich eine vorgegebene Schwenkbewegung des entsprechenden Körperteils um das zu vermessende Gelenk, die zumindest um einen vorgegebenen Winkel zu erfolgen hat. Die erfindungsgemäße Messvorrichtung ist ferner zur Bereitstellung der Werte in maschinell weiterverarbeitbarer Form eingerichtet, um auch die Ersteinstellung von Fitnessgeräten auf die ermittelte Höhe des Drehpunkts des Gelenks der jeweiligen Person zu automatisieren.

Mit der erfindungsgemäßen Messvorrichtung wird somit erstmals eine Vorrichtung bereitgestellt, mit der vor Beginn eines Trainingsprogramms eine automatisierte Ersteinstellung der Fitnessgeräte auf die entscheidenden Körpergeometriemerkmale der jeweiligen Person möglich ist, nämlich auf eine oder mehrere Gelenkdrehachsen der jeweiligen Person bzw. auf die Höhe des Drehpunkts des jeweiligen Gelenks der jeweiligen Person.

Neben der Ersteinstellung von Fitnessgeräten auf die Körperkraft ist die Ersteinstellung auf die Körpergeometrie des jeweiligen Probanden entscheidend für den Trainingserfolg. Was bisher immer unter Anleitung eines fachkundigen Therapeuten erfolgen musste oder, falls sie von dem Übenden selbst vorgenommen wurde, eine hohe Fehlerquelle darstellte, kann mit der erfindungsgemäßen Messvorrichtung erstmals automatisiert allein durch Ausführen einer vordefinierten Bewegung erfolgen, so dass Personalkosten gespart und eine Fehlerquelle ausgeschlossen werden kann.

Neben der Kostenersparnis für die wegfallenden Trainer- bzw. Therapeutenarbeitsstunden wird damit auch die Hemmschwelle für den Trainierenden gesenkt, ein bestimmtes Training aufzunehmen, da auf einfache und schnelle Weise und ohne fremdes Zutun eine Ersteinstellung der beteiligten Fitnessgeräte möglich ist.

Die erfindungsgemäße Messvorrichtung kann beispielsweise in einem Fitnesscenter, einer Reha-Einrichtung oder einem Fitnessraum eines Hotels oder dergleichen eingesetzt werden, um für jede Testperson personenbezogene Einstell-Parameter der vorhandenen Fitnessgeräte zu ermitteln. In diesem Sinne dient die Messvorrichtung zum automatisierten Ermitteln beispielsweise einer Armeinstellung und/oder Sitz-Schultergelenk-Abstandseinstellung eines oder mehrerer Fitnessgeräte, beispielsweise einer elektronisch gesteuerten Zug- oder Stemmeinrichtung zum Training der Brust- oder Armmuskulatur, wobei es entscheidend auf den für die jeweilige Person passenden Abstand des von der Testperson zu ergreifenden Übungsorgans von der Sitzhöhe und dem Schultergelenk ankommt.

Vorteilhaft weist die Messvorrichtung dazu eine Personenaufnahme auf, mit der eine vorgegebene Haltung der Testperson vordefinierbar ist, beispielsweise einen Sitz mit einer Sitzfläche und einer Rückenlehne, im dem die jeweilige Person Platz nehmen kann, wobei die vorgegebene Haltung dann eine sitzende Haltung ist. Weiterhin weist die Messvorrichtung vorteilhaft ein Vorgabemittel zum Vorgeben zumindest eines erfassbaren Parameters auf, welcher einer Position des mit der vorgegebenen bzw. standardisierten Körperbewegung bewegten Körperteils entspricht. Ferner ist vorteilhaft eine Erfassungseinrichtung zum Erfassen von zumindest zwei Werten vorgesehen - einem zu Beginn des vorgegebenen Winkels und einen am Ende des vorgegebenen Winkels - zumindest eines Parameters eingerichtet, wobei der Parameter einer Position des mit der vorgegebenen Körperbewegung um das gelenkbewegten Körperteils bei der Ausführung der vorgegebenen Körperbewegung entspricht.

Mit dieser Anordnung ist zunächst eine ortsfeste Haltung des zu vermessenden Gelenks sichergestellt, da die zu vermessende Person in der Personenaufnahme in der vorgegebenen Haltung aufgenommen ist. Ferner wird über das Vorgabemittel sichergestellt, dass die standardisierte Körperbewegung in zumindest einen erfassbaren Parameter transformiert wird. Vorteilhaft ist das Vorgabemittel dabei gleichzeitig so eingerichtet, dass sichergestellt wird, etwa über eine entsprechende mechanische Führung der Körperbewegung oder über einen Antrieb zur passiven Ausführung der Körperbewegung, dass die von der zu vermessenden Person ausgeführte Bewegung der gewünschten, standardisierten Körperbewegung um das Gelenk entspricht.

Dadurch, dass von der Erfassungseinrichtung an zwei Positionen der vorgegebenen Körperbewegung Werte erfasst werden, wobei die beiden Positionen durch einen vorgegebenen Winkel voneinander getrennt sind, und wobei die ortsfeste Lage des zu vermessenden Gelenks und die korrekte Ausführung der gewünschten Körperbewegung sichergestellt sind und beide Werte einer Position auf einer Bewegungsbahn um das Gelenk entsprechen, kann die Lage, insbesondere die Höhenlage des Drehpunkts des Gelenks ermittelt werden, welche dadurch, dass die Personenaufnahme eine bestimmte Haltung der zu vermessenden Person vorgibt, in Bezug zu dieser Haltung oder zu einem Fixpunkt an der Personenaufnahme gesetzt werden kann. Es ist somit beispielsweise möglich, eine Schultergelenkshöhe bezogen auf eine bestimmte Sitzhöhe bei den zu vermessenden Personen zu ermitteln, was eine entscheidende Eingangsgröße bei der Einstellung von Oberkörper-Trainingsgeräten darstellt.

Vorteilhaft weist die Messvorrichtung ferner eine Recheneinrichtung auf, welche Mittel zum Berechnen des Gelenkdrehpunkts aus den erfassten Parameterwerten umfasst, so dass als Ausgangsgröße direkt die gewünschte Gelenkdrehpunkthöhe oder eine entsprechende Fitnessgeräteeinstellung entnommen werden kann.

Als Teil des Vorgabemittels ist vorteilhaft ein gegenüber der Personenaufnahme lediglich mit Bewegungsfreiheitsgrad in Richtung der durchzuführenden Körperbewegung angeordnetes Angriffsorgan vorgesehen, welches mit dem jeweiligen um das zu vermessende Gelenk schwenkbaren Körperteil angreifbar ist, um so eine geführte Ausführung der vorgegebenen Körperbewegung durch die jeweilige Person sicherzustellen. Dabei weist das Angriffsorgan gegenüber der Personenaufnahme jedoch eine solche Bewegungsfreiheit auf, dass Testpersonen unterschiedlicher Körpergeometrie die standardisierte Körperbewegung ausführen können, also beispielsweise Personen unterschiedlicher Größe und entsprechend unterschiedlich hoch gelegenen Schultergelenksdrehpunkten und unterschiedlich langen Armen eine Armschwenkung um das Schultergelenk, so dass sich die Führung lediglich auf eine Führung gegen Abweichungen zur Körperseite hin beschränkt. Vorteilhaft ist daher eine Anzeige vorgesehen, vorzugsweise eine Benutzerführung auf einem Bildschirm, mit der die jeweilige Person zur Durchführung der standardisierten Bewegung instruiert wird.

Zur Vorgabe zumindest eines erfassbaren Parameters, der eine Position des mit der vorgegebenen Körperbewegung um das Gelenk bewegten Körperteils wiedergibt, müssen die Vorgabemittel so geschaltet sein, dass sie die Position des Körperteils auf eine maschinenlesbare Skala übertragen.

Dazu ist prinzipiell eine mechanische Verbindung eines ortsfest an dem Körperteil angebrachten Trägerorgans oder eines mit dem um das zu vermessende Gelenk schwenkbare Körperteil angreifbaren Angriffsorgans denkbar, wobei sich die Analogie zwischen der Position des Körperteils und dem diese wiedergebenden Parameter aus der Auslenkung bzw. Verschiebung der mechanischen Verbindung durch die Schwenkbewegung um das Gelenk ergibt. Das Vorgabemittel besteht in diesem Fall aus dem Angriffs- oder Trägerorgan und seiner mechanischen Verbindung mit einem Geräterahmen oder dergleichen. Es wäre jedoch ebenfalls denkbar, anstatt einer mechanischen Verbindung des zu verschwenkenden Körperteils unter dem nötigen Bewegungsfreiheitsgrad mit einem Festpunkt und einem Erfassen der Verdrehung oder Verschiebung der mechanischen Verbindung, am zu verdrehenden Körperteil ein Trägerorgan, beispielsweise einen Handschuh oder ein Armband vorzusehen, welches einen Positionssensor trägt, beispielsweise einen mit einer Lichtabtasteinrichtung erfassbaren Reflektor oder einen elektromagnetischen Positionssensor, so dass ein Parameter erfasst werden kann, der direkt und ohne Umrechnung die Position des Körperteils während der Bewegung wiedergibt, wobei die Führung der Bewegung dann per Bildschirm-Benutzerführung erfolgen könnte oder durch ein ergänzend vorgesehenes Angriffsmittel mit mechanischer Führung.

Besonders vorteilhaft ist als mechanische Verbindung eine Schwenkhebelanordnung mit zwei parallel zur Gelenkdrehachse verlaufenden Schwenkachsen, die einenends mit dem zu verschwenkenden Körperteil mit bewegt wird und anderenends ortsfest gegenüber der Personenaufnahme angeordnet ist. Die Verdrehung der beiden Schwenkhebel um die beiden Schwenkachsen bildet somit zwei Parameter, die in Beziehung zu der Position des um das Gelenk verschwenkten Körperteils stehen. Weiterhin wäre auch eine Kombination aus einer Linearführung und einem Schwenkhebel denkbar, wobei sich die Position des Körperteils dabei aus einer Verschwenkung des Schwenkhebels um eine zu der Gelenkachse parallelen Schwenkachse und einer Linearverschiebung der Linearführung ergibt.

Gemäß einer bevorzugten Ausführungsform umfasst das Vorgabemittel ein Angriffsorgan in Form von zwei Griffen, die jeweils über eine Schwenkhebetanordnung mit der Rückenlinie eines die Personenaufnahme bildenden Sitzes verbunden sind, wobei die Schwenkhebelanordnung einen ersten schwenkbar an der Rückenlehne angelenkten Schwenkhebel und einen zweiten an dem anderen Ende des ersten Schwenkhebel angelenkten Schwenkhebel umfasst und der Griff an dem anderen Ende des zweiten Schwenkhebels vorgesehen ist. Die Erfassungseinrichtung weist in dieser bevorzugten Ausführungsform eine erste Winkelerfassungseinrichtung zum Erfassen eines Schwenkwinkels des ersten Schwenkhebels um den Anlenkpunkt an der Rückenlehne und eine zweite Winkelerfassungseinrichtung zum Erfassen des Schwenkwinkels des zweiten Schwenkhebels um das Ende des ersten Schenkhebels auf. Dabei beträgt der vorgegebene Winkel, um den die Arme um das Schultergelenk geschwenkt werden müssen, vorzugsweise 180°. Wenn die Armschwenkbewegung ordnungsgemäß mit gestreckten Armen ausgeführt wird, lässt sich aus den zu Beginn der Schwenkbewegung und am Ende der Schwenkbewegung erfassten Winkelwerten an der ersten und der zweiten Schwenkachse die Höhe des Gelenkdrehpunkts errechnen.

Zur Erfassung des Hüftgelenkdrehpunkts wäre es dagegen denkbar, die Rückenlehne des Sitzes schwenkbar an der Sitzfläche zu befestigen und einen linear auf der Rückenlehne geführten Schlitten an einem festen Punkt mit dem Oberkörper der betätigenden Person zu verbinden, beispielsweise über einen Brustgurt. Bei Ausführung einer Rumpfschwenkung um das Hüftgelenk kann dann die Verdrehung der Rückenlehne gegenüber der Sitzfläche und die lineare Verschiebung des körperfest angebrachten Brustgurts auf der Rückenlehne erfasst werden, woraus sich dann der Hüftgelenksdrehpunkt errechnen lässt, wenn die Rumpfschwenkung um einen vorbestimmten Winkel ausgeführt wird, bzw. wenn die Erfassung der Verschwenkung der Rückenlehne über der Sitzfläche und der Linearverschiebung des körperfest angebrachten Brustgurts auf der Rückenlehne an zwei festgelegten Winkeln der Rumpfschwenkung erfolgt.

Auf gleiche Weise kann die Höhe des Kniegelenks erfasst werden, indem das Angriffsmittel beispielsweise als Fußschlaufe ausgestaltet ist, welche über eine gleichartige Schwenkhebelanordnung mit einem ortsfesten Punkt verbunden ist und wiederum die Schwenkbewegung des Unterschenkels um das Kniegelenk um einen festgelegten Winkel erfolgt, wobei die Erfassung der Drehwinkel in der Schwenkhebelanordnung zu Beginn der vorgegebenen Unterschenkelverschwenkung und am Ende der vorgegebenen Unterschenkelverschwenkung erfolgt.

Selbstverständlich ist es möglich, die vorstehend beschriebenen Ausführungsformen zu kombinieren.

Vorteilhaft ist die Personenaufnahme als Sitz mit einem höhenverstellbaren Stuhlbein ausgestattet, wobei Mittel zum Vorgeben einer festgelegten Knieabwinklung über die Höhenverstellung des Stuhlbeins vorgesehen sind, welche insbesondere motorisch bzw. programmgesteuert erfolgt. Mit dieser Anordnung gelingt es, eine Beineinstellung für Fitnessgeräte zu ermitteln, da es für eine Beineinstellung von Fitnessgeräten zumeist optimal ist, wenn die Sitzhöhe so gewählt ist, dass die Beine im rechten Winkel abgewinkelt auf dem Boden aufsetzen.

Die Mittel zum Vorgeben der festgelegten Knieabwinkelung über die Höhenverstellung können dabei vorteilhaft eine Fußauflage-Kontaktplatte umfassen, die zur Abgabe eines Schaltsignals zum Anhalten der Höhenverstellung des Stuhlbeins bei aufsetzendem bzw. abhebendem Fuß eingerichtet ist. Wenn der Fuß bei nach unten verfahrenden Stuhlbeinen auf die Kontaktplatte aufsetzt, schaltet die Höhenverstellung des Stuhlbeins somit ab, so dass die Knieabwinklung genau den gewünschten 90° entspricht. Das gleiche gilt, wenn die Höhenverstellung auf dem Weg nach oben abschaltet, wenn der Fuß gerade von der Kontaktplatte abhebt.

Vorteilhaft ist dabei ferner eine Starteinrichtung zum Start der Höhenverstellung des Stuhlbeins vorgesehen, mit der die zu vermessende Person die Höhenverstellung auslösen kann, z. B. ein Druckknopf oder eine auf einem Touchscreen laufende, interaktive Benutzerführung. Besonders vorteilhaft ist es dabei, wenn die Höhenverstellung nach oben auslöst, wenn an der Kontaktplatte erfasst wird, dass der Fuß aufsetzt, und nach unten auslöst, wenn an der Kontaktplatte erfasst wird, dass der Fuß nicht auf der Kontaktplatte aufsetzt. Dazu kann die Fußauflage-Kontaktplatte mit einem Endschalter verbunden sein, welcher bei aufsetzendem bzw. abhebendem Fuß einen Antrieb für die Höhenverstellung des Stuhlbeins abschaltet. Alternativ dazu kann die Fußauflage-Kontaktplatte mit einer Kontaktfolie versehen sein, die bei Druckkontakt einen besseren Stromdurchgang aufweist. Ebenfalls denkbar wäre eine Lichtschrankenanordnung.

Vorteilhaft ist ferner eine Sitzhöhen-Erfassungseinrichtung vorgesehen, die zum Erfassen der Sitzhöhe bei der festgelegten Knieabwinklung, also bei Erhalt des Schaltsignals von der Fußauflage-Kontaktplatte eingerichtet ist, insbesondere mit einer Lichtschranke bzw. einem Lichtabtaster und einer von der Lichtschranke abgetasteten Inkrementalskala.

Aus der Sitzhöhe kann vorteilhaft nicht nur ein Beineinstellungsparameter des Fitnessgeräts bzw. eine Unterschenkelabmessung der Testperson errechnet werden, sondern vorteilhaft auch eine Gesamtbeinlänge oder eine entsprechende Einstellung des Fitnessgeräts abgeschätzt werden, etwa ein Abstand von Pedalen zur Sitzhöhe. Dazu kann auf bekannte Verhältnisse von Unterschenkellänge zur Oberschenkellänge zurückgegriffen werden, die bei den meisten Menschen mit sehr guter Näherung eingehalten werden.

Vorteilhaft kann bei der Abschätzung der Gesamtbeinlänge aus der Sitzhöhe bei der festgelegten Knieabwinklung jedoch zusätzlich auf die ermittelte Höhe des Schultergelenks zurückgegriffen werden oder auf die ermittelte Höhe des Hüftgelenkdrehpunkts.

In der Ausführungsform der Meßvorrichtung als Meßstuhl dient der höhenverstellbare Sitz somit zur Vorgabe der vorzugebenden Haltung, nämlich einer Sitzhöhe bei 90°-Knieabwinklung, wobei das Vorgabemittel zwei über eine Schwenkhebelanordnung mit der Rückenlehne des Sitzes verbundene Griffe zum beidarmigen Ausführen der Armschwenkung aufweisen kann. Zur Ermittlung der Schultergelenkshöhe in der sitzenden Position mit der 90°-Knieabwinklung ist es dabei ferner vorteilhaft, wenn die Recheneinrichtung Mittel zum Errechnen einer Höhe des Schultergelenkdrehpunkts in einer stehenden Haltung oder zur Berechnung einer entsprechenden Einstellung des Fitnessgeräts aufweist, wobei auf die ermittelte Höhe des Schultergelenk- Drehpunkts in der sitzenden Haltung mit der festgelegten Knieabwinklung und auf die ermittelte bzw. geschätzte Standbeinlänge oder die ermittelte Sitzhöhe als Eingangsgrößen zurückgegriffen werden kann.

Dabei ist es ferner vorteilhaft, wenn die durch die Gelenkhebelanordnung erzeugten Drehwinkelwerte an den beiden horizontalen Achsen der Gelenkhebelanordnung ferner dazu verwendet werden, die Armlänge zu berechnen.

Weiterhin kann als Startpunkt für die Armschwenkung um das Schultergelenk eine vertikale Armhaltung definiert wird, da diese von der Testperson auf einfache Weise eingenommen werden kann, indem sie seine Arme baumeln lässt. Als vorbestimmter Winkel kann 180° gewählt werden.

Im Sinne einer vollständigen Erfassung aller für die Einstellung von Fitnessgeräten relevanten Körpermerkmalen ist es ferner vorteilhaft, wenn die erfindungsgemäße Messvorrichtung mit zumindest einem Kraftaufnehmer ausgestattet ist, mit dem eine von der jeweiligen Person aufgebrachte Krafteingabe erfasst werden kann. Besonders vorteilhaft ist dabei ein Kraftaufnehmer zuschaltbar in die Anlenkung des ersten Gelenkhebels integriert und ein zweiter Kraftaufnehmer in die Anlenkung des zweiten Gelenkhebels, so dass bei Zuschaltung der Kraftaufnehmer das Vorgabemittel nicht nur zur Erzeugung von zu der Körpergelenkhöhe analogen Parameterwerten genutzt werden kann, sondern ebenfalls zur Kraftmessung, wenn die Gelenkhebelanordnung in einer definierten Stellung durch Zuschaltung der Kraftaufnehmer arretiert wird, so dass die Testperson mit der gewünschten Muskelgruppe, beispielsweise Bizeps oder Trizeps eine Krafteingabe auf die Schwenkhebelanordnung aufbringt und diese in dazu analogen Werten an den Kraftaufnehmern erfasst werden kann.

Vorteilhafte Weiterbildung erfassen weitere Kraftaufnehmer, beispielsweise über ein Brustpolster, so dass mit einer Beuge des Oberkörpers nach vorne eine Kraft auf das Brustpolster aufgebracht werden kann, oder einen in die Anlenkung der Rückenlehne an der Sitzfläche integrierten Kraftaufnehmer, so dass bei einer Streckung des Oberkörpers nach hinten eine Kraft auf diesen Kraftaufnehmer aufgebracht werden kann. Ferner können weitere körpereigene Merkmale erfasst werden, beispielsweise das Gewicht über eine Waage oder der Puls der Testperson über einen Pulsmesser, welche zur Berechnung eines Trainingsprogramms verwendet werden können, auf welches wiederum die Fitnessgeräte eingestellt werden.

Vorteilhaft hat die Recheneinrichtung der Messvorrichtung darüber hinaus eine Speichereinrichtung zum Speichern der ermittelten Körpergeometrie- bzw. -kraft- bzw. sonstigen körperbezogenen Parameterwerte, der dazu analogen Parameter oder der ermittelten Fitnessgeräte-Einstellparameter bzw. des Trainingsprogramms auf eine Speicherkarte, die von der vermessenen Testperson mitgeführt werden kann und an den einzelnen Fitnessgeräten in einen entsprechenden Kartenleser eingegeben werden kann, woraufhin sich das jeweilige Fitnessgerät auf die auf der Karte enthaltenen Werte einjustiert. Alternativ dazu kann auch eine Vernetzung der Messvorrichtung mit den einzelnen Fitnessgeräten vorgesehen sein.

Vorteilhaft wird somit ein Messstuhl geschaffen, an dem alle oder zumindest viele Einstellparameterwerte für eine Vielzahl verschiedener Fitnessgeräte ermittelt werden können, und zwar aus unter vordefinierten Bedingungen ausgeführten Schwenkbewegungen um ortsfest gehaltene Körpergelenke und aus anderen Eingaben, wie beispielsweise Körperkrafteingaben oder Körpergewichtseingaben.

Die vorhergehend beschriebenen und beanspruchten Ausführungsformen lassen sich dabei, soweit es sinnvoll erscheint, auf jedwede Art kombinieren, ohne den Rahmen der Erfindung zu verlassen.

Im Folgenden wird eine bevorzugte Ausführungsform der Erfindung anhand der beiliegenden Zeichnungen erläutert. Es zeigen:
- Figur 1: eine Seitenansicht einer als Messstuhl ausgebildeten Ausführungsform der Erfindung;
- Figur 2: eine Frontansicht des in Figur 1 gezeigten Messstuhls;
- Figur 3: eine Draufsicht auf eine weitere, als Messstuhl ausgebildete Ausführungsform;
- Figur 4: eine Seitenansicht des in Figur 4 gezeigten Messstuhls;
- Figur 5: eine Frontansicht des in Fig. 4 und 5 gezeigten Messtuhls; und
- Figur 6: ein Blockschaubild zur Veranschaulichung der Funktionsweise des in den vorhergehenden Figuren gezeigten Messstuhls.

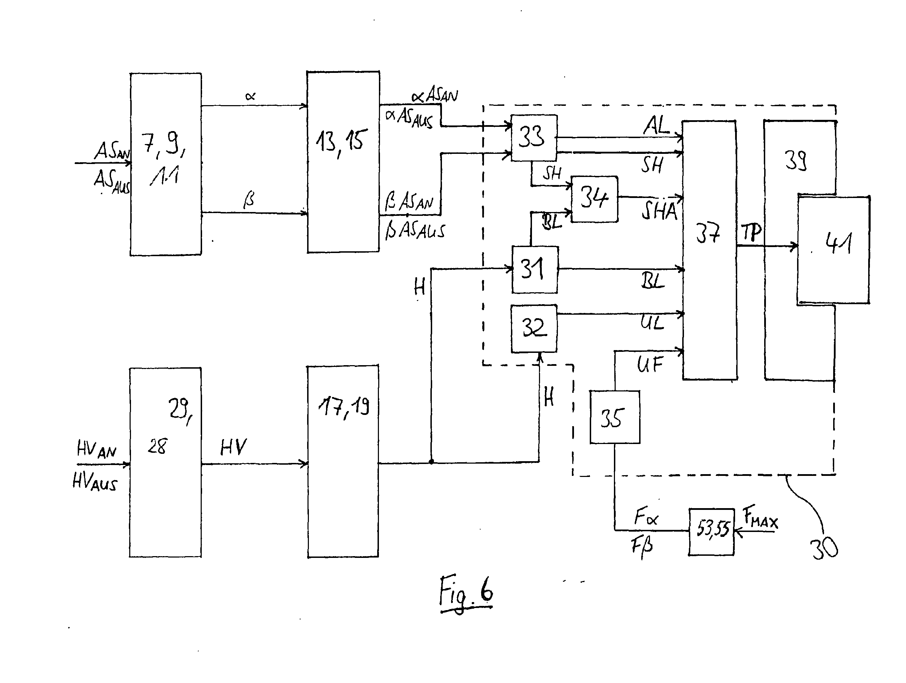

Wie dabei insbesondere aus dem Blockschaubild der Figur 6 deutlich wird, welches auch als Ablaufdiagramm gedeutet werden kann, können die im Rahmen der Ansprüche vorrichtungsmäßig beanspruchten Merkmale der Erfindung auch als Verfahrensmerkmale ausgesehen werden.

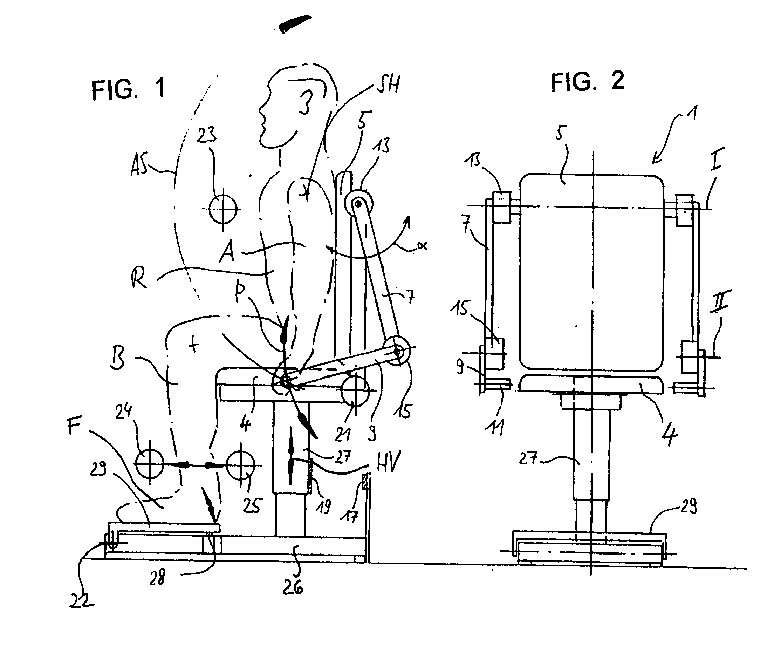

In den Figuren 1 und 2 ist mit 1 eine als Stuhl ausgebildete Ausführungsform der erfindungsgemäßen Messvorrichtung bezeichnet. Der Messstuhl 1 weist ein Rahmengestell mit einer auf einem Stuhlbein 27 gelagerten Sitzfläche 4 auf, an die eine Rückenlehne 5 angelenkt ist, wobei das Stuhlbein 27 höhenverstellbar ist, wie durch den mit HV bezeichneten Doppelpfeil angedeutet ist, und wobei die Sitzfläche 4 und die Rückenlehne 5 gepolstert sind und zusammen einen Sitz 4, 5 bilden. Der Sitz 4, 5 dient hier als Personenaufnahme 4, 5.

An der Rückenlehne 5 ist eine Gelenkhebelanordnung mit zwei Gelenkhebeln 7, 9 angelenkt, wobei der erste Gelenkhebel 7 um eine erste horizontale Achse I schwenkbar an der Rückenlehne 5 angelenkt ist und der zweite Gelenkhebel 9 um eine zweite horizontale Achse II an dem anderen Ende des ersten Gelenkhebels 7. Dabei ist die Gelenkhebelanordnung sowohl auf der linken als auch auf der rechten Seite des Messstuhls 1 vorgesehen, so dass die zu vermessende Person - wie in Figur 1 angedeutet - die Gelenkhebelanordnung mit beiden Armen jeweils an einem Griff 11 angreifen kann, welcher am dem dem Anlenkpunkt an der Rückenlehne 5 entgegengesetzten Ende der Gelenkhebelanordnung vorgesehen ist und hier als Angriffsorgan 11 dient.

Der Messstuhl 1 bzw. das höhenverstellbare Stuhlbein 27 ist dabei auf einer Bodenplatte 26 gelagert. An der Bodenplatte 26 ist über ein frontseitiges Scharnier 22 eine Fußauflage-Kontaktplatte 29 schwenkbar gelagert. Ein von dem Fuß F der zu vermessenden Person auf die Bodenplatte 26 beim Ablassen der Sitzfläche 4 ausgeübter Druck wird dabei auf einen im rückwärtigen Bereich der Fußauflagen-Kontaktplatte 29 aufgebrachten Endschalter 28 übertragen, wenn der Fuß F aufsetzt.

Zum Erfassen der momentanen Sitzhöhe ist dabei eine am Stuhlbein 27 angebrachte Inkrementalskala 19 vorgesehen, die mit einem von einer Lichtschrankeneinrichtung 17 bzw. einem Lichtstrahlgeber mit angeschlossenen Inkrementenzähler abgetastet wird. Ferner ist in die Anlenkung des ersten Gelenkhebels 7 der Gelenkhebelanordnung an die Rückenlehne 5 ein inkrementeller Winkelgeber 13 integriert und in die Anlenkung des zweiten Gelenkhebels 9 an den ersten Gelenkhebel 7 ein zweiter inkrementeller Winkelgeber 15, mit denen eine Verdrehung der Gelenkhebelanordnung um die erste horizontale Achse I und die zweite horizontale Achse II bei einer Armschwenkbewegung AS um einen Schultergelenk-Drehpunkt SH erfasst werden kann, wobei die Verdrehung um die erste horizontale Achse I mit α und die Verdrehung um die zweite horizontale Achse mit β bezeichnet ist. Die Winkelgeber 13, 15 dienen hier als Erfassungseinrichtung 13, 15 im Sinne von Anspruch 2.

Weiterhin weist der Messstuhl 1 verschiedene Kraftaufnehmer auf, mit denen die Körperkraft der zu vermessenden Person bzw. unterschiedliche Muskelgruppen der zu vermessenden Person erfasst werden kann. Einerseits ist in die Anlenkung des ersten Gelenkhebels 7 an die Rückenlehne 5 neben dem inkrementellen Winkelgeber 13 ein erst in Fig. 6 bezeichneter Kraftaufnehmer-Verdrehwiderstand, nämlich eine Kraftmessdose 53, integriert und in die Anlenkung des zweiten Gelenkhebels 9 an den ersten Gelenkhebel 7 ein weiterer Kraftaufnehmer-Verdrehwiderstand 55. Ferner ist die Rückenlehne 5 über einen Kraftmesser 21 an der Sitzfläche 4 angelenkt. Weiterhin ist eine Brustpolsterrolle 23 vor der Brust des Trainierenden über einen zuschaltbaren Kraftaufnehmer anbringbar. Weitere, jeweils über einen zuschaltbaren Kraftaufnehmer schwenkbar angeordnete Polsterrollen 24, 25 dienen dagegen der Erfassung der Körperkraft an den Adduktoren und Abduktoren der Beine B der zu vermessenden Person.

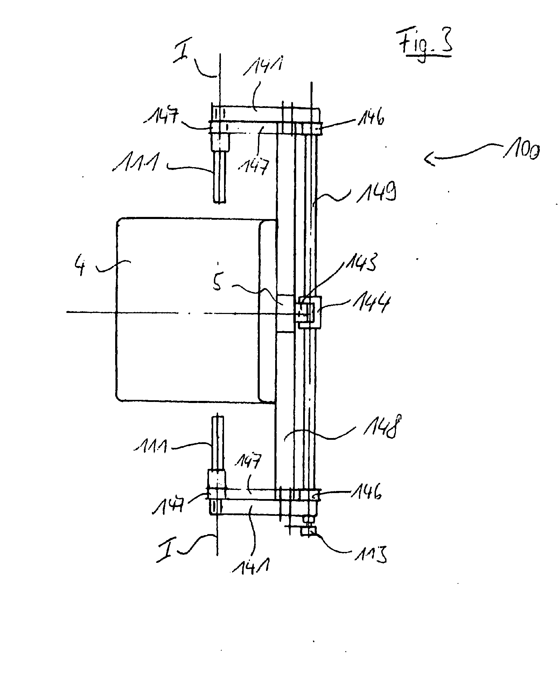

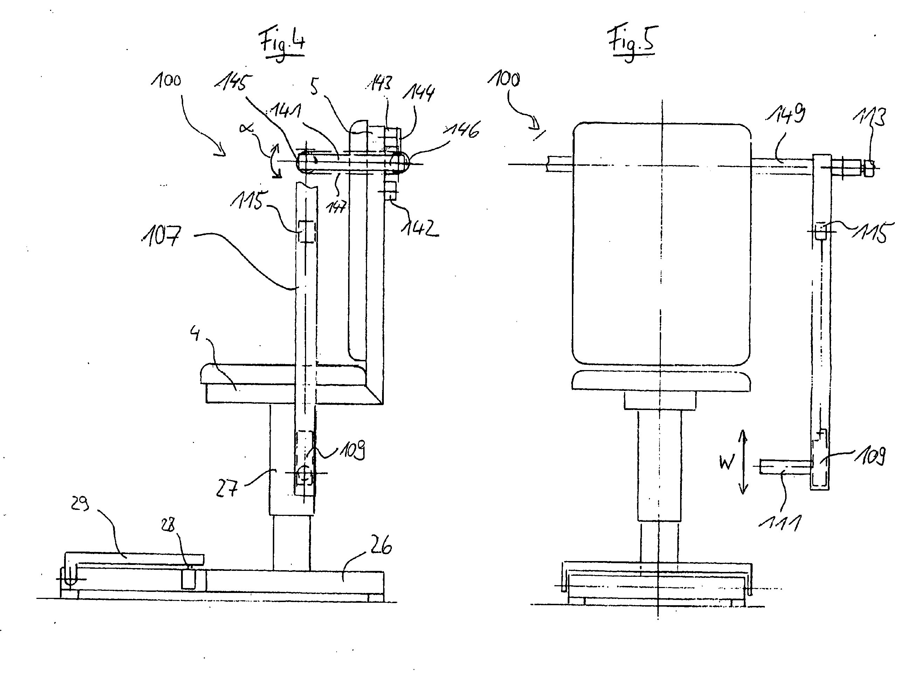

Im Folgenden wird anhand der Figuren 3 bis 5 eine weitere Ausführungsform der Erfindung erläutert. Dabei sind gleiche Bauteile mit den gleichen Bezugszeichen wie in den Figuren 1 und 2 bezeichnet und Bauteile, die denjenigen der Figur 1 und 2 funktional entsprechen mit Bezugszeichen, die um 100 hoch gezählt wurden.

Der Messstuhl 100 weist dabei ebenfalls ein Rahmengestell mit einer auf einem Stuhlbein 27 gelagerten Sitzfläche 4 auf, wobei jedoch die Rückenlehne 5 starr mit der Sitzfläche 4 verbunden ist. Dabei ist das Stuhlbein 27 ebenfalls höhenverstellbar und auf der Bodenplatte 26 gelagert, welche wiederum die Fußauflagenkontaktplatte 29 mit dem Endschalter 28 trägt.

Das Vorgabemittel zum Vorgeben von erfassbaren Parametern, die einer Position des mit der vorgegebenen Körperbewegung um das Schultergelenk bewegten Arms bzw. der um das Schultergelenk bewegten Arme entsprechen, hat jedoch einen von der ersten Ausführungsform abweichenden Aufbau. Dabei ist mit 107 ein um eine erste horizontale Achse I angelenktes Führungsrohr bezeichnet, in dem ein mit einem jeweiligen Griff 111 verbundener Schlitten 109 linear geführt ist. Der Anlenkpunkt des Führungsrohrs 107 befindet sich dabei jeweils an einem nur in Figur 3 dargestellten Holm 141, welcher mit einem ebenfalls nur dort gezeigten Träger 148 mit dem Rahmengestell des Sitzes 4, 5 verbunden ist. Der Holm 141 verläuft dabei in horizontale Richtung und erstreckt sich ungefähr auf der zu erwartenden Höhe eines Schultergelenks einer Testperson vom rückwärtigen Bereich des Sitzes soweit nach vorne, dass der Anlenkpunkt des Führungsrohrs 107 in etwa in die Nähe der Drehachse der von der Testperson um das Schultergelenk durchzuführenden Armschwenkbewegung gelangt. Dadurch wird der Verfahrweg des Haltegriffs 111 bzw. des Schlittens 109 in dem Führungsrohr 107 so weit als möglich beschränkt, was insbesondere in der waagrechten Stellung des Führungsrohrs 107 von Vorteil ist.

Dabei wird der Verfahrweg W des Schlittens 109 von in dem Führungsrohr 107 untergebrachten Positionssensoren 115 erfasst. Die Erfassung der Schwenkbewegung des Führungsrohrs 107 könnte dabei direkt am Anlenkpunkt des Führungsrohrs 107 an dem Holm 141 erfolgen. Vorteilhaft ist jedoch eine gemeinsame Welle 149 vorgesehen, die mit beiden Armgriffen 111 bzw. Führungsrohren 107 in drehschlüssiger Verbindung steht, so dass eine Synchronisierung der Armbewegungen erfolgt, wobei die Erfassung der Schwenkbewegung an der Synchronisierungswelle 149 erfolgt, im dargestellten Beispiel über ein Potentiometer 113. Zur Übertragung der Schwenkbewegung des Führungsrohrs 107 auf die Synchronisierungswelle 149 ist dabei jeweils ein Zahnriemenband 147 vorgesehen, welches einerseits über eine auf der Synchronisierungswelle 149 angebrachte Riemenscheibe 146 und andererseits über eine am Anlenkpunkt mit dem Führungsrohr 107 verbundene Riemenscheibe 145 geführt ist.

Die Synchronisierungswelle 149 ist dabei beidseitig in dem jeweiligen Holm 141 gelagert und weist mittig einen Dorn 144 auf, mit dem die Drehbewegung der Synchronisierungswelle 149 an dafür an der Rückenlehne 5 vorgesehenen Anschlägen 142, 143 auf den gewünschten Winkel von 180° begrenzt wird.

Die Funktionsweise der erfindungsgemäßen Messvorrichtung soll im Folgenden am Beispiel des Messstuhls 1 unter Bezugnahme auf die Figuren 1 bis 3, 6 erläutert werden. Das durch die Gelenkhebelanordnung mit dem ersten und dem zweiten Gelenkhebel 7, 9 und das Angriffsorgan 11 gebildete Vorgabemittel 7, 9, 11 dient dabei der Erzeugung der beiden Parameter α, β, nämlich die Verdrehung des ersten Gelenkhebels 7 um die erste horizontale Achse I (= α) und des zweiten Gelenkhebels 9 um die zweite horizontale Achse II (= β) bei einer vorgegebenen Armschwenkung AS der zu vermessenden Person. Die beiden Parameter α, β werden dabei zu Beginn AS_{AN} der Armschwenkbewegung AS um einen festgelegten Winkel und am Ende AS_{AUS} dieser Schwenkbewegung durch die Winkelgeber 13, 15 erfasst. Aus den erhaltenen beiden Werten α AS_{AN}, α AS_{AUS}, β AS_{AN}, β AS_{AUS} der beiden Parameter α, β wird dann mit einer entsprechenden Software 33 auf einer Recheneinrichtung 30 eine Höhe des Schultergelenk-Drehpunkts SH errechnet, wobei die Berechnung bei einer bekannten Sitzhöhe und damit einer bekannten Höhe des Anlenkpunkts der Gelenkhebelanordnung 2 an der Rückenlehne 5 erfolgt. Ferner wird dabei eine Armlänge AL der zu vermessenden Person berechnet.

Bei dem Messstuhl 100 dient analog das angelenkte Führungsrohr 107 mit dem am darin geführten Schlitten 109 befestigten Griff 111 zur Erzeugung des Wegs W und der Verdrehung α.

Aus dem vorstehend Gesagten wird deutlich, dass neben einem mechanisch arbeitenden Parameter-Vorgabemittel, wie es die Gelenkhebelanordnung 7, 9 bzw. 107, 109 darstellt, auch ein beispielsweise optisch oder elektromagnetisch arbeitendes Vorgabemittel denkbar wäre, bei dem wie in den dargestellten Beispielen durch die Griffe 11; 111 sichergestellt ein entsprechender Sensor beispielsweise mit einem Armband an einer festgelegten Stelle des Arms mit der zu vermessenden Person verbunden ist. Dabei kann der die Position des mit der Armschwenkung AS um das Schultergelenk bewegten Arms A wiedergebende Parameter ein entsprechender Parameter im Sensorkoordinatensystem sein, wobei auch hier die Armschwenkung AS um einen festgelegten Winkel ausgeführt werden muss. In diesem Fall wäre es auch möglich, den Sensor mit dem Oberarm der zu vermessenden Person zu verbinden. Denn bei der dargestellten Ausführungsform ist es für eine korrekte Erfassung der Höhe des Schultergelenkdrehpunkts nötig, dass die Armschwenkung AS mit durchgestrecktem Arm A ausgeführt wird, was sich durch einen am Oberarm angebrachten Sensor als Fehlerquelle ausscheiden ließe.

Weiterhin wäre es beispielsweise möglich, neben dem Vorgabemittel 7, 9, 11 bzw. 107, 109, 111 ein entsprechendes Vorgabemittel zur Erfassung einer Höhe des Kniegelenkdrehpunkts vorzusehen, beispielsweise eine - oder zwei - Fußschlaufen, die über eine der Gelenkhebelanordnung 7, 9 entsprechende Gelenkhebelanordnung an einem festen Punkt angelenkt ist, so dass sich über den Winkelinkrementgeber 13, 15 entsprechende Inkrementgeber eine Höhe des Kniegelenks bei bekannter Sitzhöhe errechnen ließe.

Ebenso wäre es denkbar, den Rumpf R der sitzenden Person mit einem entsprechenden Brustgurt auf einem in einer entlang der Rückenlänge 5 vertikal verlaufenden Linearführung geführten Schlitten zu fixieren, so dass über eine bei flexibler Anlenkung der Rückenlehne 5 an der Sitzfläche 4 durchgeführten Rumpfschwenkung um einen vorgegebenen Winkel und Erfassung der Linearverschiebung des Schlittens und der Abwinklung der Rückenlehne 5 gegenüber der Sitzfläche 4 eine Lage des Hüftgelenks bei vorgegebener Sitzlage ermittelt werden kann.

Um mit der festgelegten Sitzhöhe einen Bezugspunkt für die Messung der Schultergelenks-Drehpunkthöhe SH bereitzustellen und die Person für die Muskelkraftmessungen in eine geeignete Sitzhaltung zu bringen, steht mit der Kontaktplatte 29 und dem Endschalter 28 ferner ein Mittel zum Vorgeben einer festgelegten Knieabwinklung bereit, d. h. ein Mittel zum Vorgeben einer Sitzhaltung mit genau vertikal ausgerichteten Unterschenkeln. Wenn die zu vermessende Person auf dem Messstuhl 1 Platz nimmt und ihre Füße F nicht auf der Fußauflagenkontaktplatte 29 aufsetzen, so dass der Endschalter 28 den Schaltkreis schließt, wird die Höhenverstellung HV des Stuhlbeins 27 nach unten betätigt, bis vom Endschalter 28 ein Schaltsignal HV_{AUS} zum Abschalten der Höhenverstellung kommt. Die Sitzhöhe in der Position mit vertikal ausgerichteten Unterschenkeln wird dann an der Sitzhöhenerfassungseinrichtung 17, 19 erfasst. Wenn die Person dagegen mit auf der Kontaktplatte 29 aufsetzenden Beinen B bzw. Füßen F Platz nimmt, wird die Höhenverstellung HV nach oben betätigt, bis der Endschalter 28 das Abschaltsignal HV_{AUS} gibt.

Aus der eingestellten Höhe HV wird dann an der Sitzhöhenerfassungseinrichtung 17, 19 die Sitzhöhe H bei vertikal ausgerichteten Unterschenkeln erfasst. Die Recheneinrichtung 30 umfasst dabei einerseits eine Einheit 32 zum Berechnen einer die Unterschenkellänge wiedergebenden Einstellung UL der einzustellenden Fitnessgeräte und andererseits eine Schätzeinrichtung 31, mit der aus der Sitzhöhe eine Gesamtbeinlänge bzw. ein die Gesamtbeinlänge wiedergebende Einstellung BL der einzustellenden Fitnessparameter umfasst. Aus der relativ zu der durch die Höhenverstellung HV festgelegten Position gemessenen Höhe SH des Schultergelenk-Drehpunkts und der aus der Sitzhöhe H in dieser Position abgeschätzten Gesamtbeinlänge BL wird mit einer weiteren Einheit 34 der Recheneinrichtung 30 eine absolute Höhe SHA des Schultergelenk-Drehpunkts errechnet, d. h. eine Höhe des Schultergelenk-Drehpunkts in aufrechter Position der zu vermessenden Person bzw. eine entsprechende Einstellung SHA der einzustellenden Fitnessgeräte.

In der festgelegten Haltung der zu vermessenden Person werden ferner Körperkraftmessungen durchgeführt. Dabei können beispielsweise Bizeps- oder Trizeps-Kraftmessungen durchgeführt werden, indem die Gelenkhebelanordnung 7, 9 in einer dafür geeigneten Ausgangsposition durch Zuschaltung von als Verdrehwiderstand in die eine oder andere Richtung wirkende Kraftaufnehmer 53, 55 gebracht wird und die zu vermessende Person beispielsweise über eine grafische Benutzerführung auf einer Bildschirmoberfläche zur Ausführung der entsprechenden Übung bzw. Aufbringung einer Maximalkraft F_{MAX} angewiesen wird.

Aus der aufgebrachten Kraft F_{MAX} resultiert jeweils eine Kraftkomponente Fα, Fβ an den beiden horizontalen Achsen I und II, die an den beiden Kraftaufnehmern 53, 55 aufgenommen wird. In einer Einheit 35 der Recheneinrichtung 30 wird daraus eine Einstellung UF der Gegengewichtserzeugungseinrichtung der Fitnessgeräte für das Bizeps- bzw. Trizepstraining erzeugt. Ergänzend kann durch Anweisung auf dem Bildschirm und entsprechende Krafterfassung auch die Körperkraft bei einer Armschwenkung in einer zu der mit AS bezeichneten Armschwenkung senkrechten Richtung erfasst werden. Weiterhin erfolgt auch die Kraftmessung an den weiteren Messpunkten durch Bildschirmanweisung an den Benutzer, eine entsprechende Kraft auf den jeweiligen Kraftaufnehmer aufzubringen, beispielsweise mit dem Rumpf R gegen die Lehne 5 oder das Brustpolster 23 zu drücken, so dass dort die Bauch- und Rückenmuskelkraft erfasst werden kann und über eine entsprechende Recheneinheit in der Recheneinrichtung 30 die Einstellung der jeweiligen Gegenkrafterzeugungseinrichtung der Fitnessgeräte errechnet werden kann.

Vorteilhaft ist ferner eine weitere Einheit 37 der Recheneinrichtung 30 vorgesehen, in der aus den einzelnen Einstellparametern, die über den Messstuhl 1 gewonnen worden sind, ein Trainingsprogramm TP erzeugt wird, wobei weitere Eingaben, wie beispielsweise das Gewicht der zu vermessenden Person (welches beispielsweise über eine in dem Messstuhl integrierte Waage erfasst worden ist), ein Ruhepuls (welcher beispielsweise über einen in den Messstuhl integrierten Pulsmesser erfasst worden ist) und ein Belastungspuls oder dergleichen in die Errechnung des Trainingsprogramms eingehen können. Über eine weitere Einheit 39 der Recheneinrichtung wird das Trainingsprogramm TP bzw. die errechneten Parameter AL, SH, SHA, BL, UL sowie UF dann auf eine Speicherkarte 41 gespeichert, mit der die vermaßte Person dann die die einzelnen Trainingsstationen seines Trainingsprogramms bildenden Fitnessgeräte durchlaufen kann.

Selbstverständlich sind Abwandlungen und Weiterbildungen möglich, ohne den Rahmen der Erfindung zu verlassen.

So wäre es beispielsweise denkbar, anstatt der Verbindungsholme 141 zwischen dem Anlenkpunkt der Führungsrohre 107 und dem Träger 148, in denen in dem in den Figuren 3 bis 5 dargestellten Beispiel auch die Synchronisierungswelle 149 gelagert ist, einen von dem Sitz 4, 5 separaten Portalaufbau der Aufhängung der Führungsrohre 107 vorzusehen.

Ebenso denkbar wäre eine Kombination einzelner Merkmale der vorhergehend beschriebenen Ausführungsformen. So könnten beispielsweise auch in der in den Figuren 3 bis 5 dargestellten Ausführungsform verschiedene Kraftaufnehmer vorgesehen sein.

Die Erfindung verkörpert sich somit in den Merkmalen des Anspruchs 1 und gemäß vorteilhafter Weiterbildungen in den Merkmalen der Unteransprüche. Folgende Merkmale, sind auch, jeweils für sich und in jedweder sinnvollen Kombination mit dem Gegenstand der Ansprüche kombinierbar:
- Punkt. 1.: Messvorrichtung, wobei das Vorgabemittel eine Antriebseinrichtung zum Antreiben des Angriffsorgans umfasst, um ein passives Ausführen der vorgegebene Körperbewegung durch die jeweilige Person zu ermöglichen.
- Punkt 2.: Messvorrichtung, wobei das Vorgabemittel und/oder die Erfassungseinrichtung mit zumindest einer interaktiven Anzeige, vorzugsweise einer interaktiven, graphischen Benutzerführung auf einem Bildschirm verbunden ist.
- Punkt 3.: Messvorrichtung 1, wobei eine Ereigniserzeugungsvorrichtung 29, 28 zum Erzeugen von Ereignissen AS_{AN}, AS_{AUS} zum Anstoßen der Positionserfassung durch die Erfassungseinrichtung 13, 15 und zum Anstoßen der Ausführung der Körperbewegung vorgesehen ist.
- Punkt 4.: Messvorrichtung 1; 100, wobei der Sitz 4, 5 ein insbesondere motorisch bzw. programmgesteuert höhenverstellbares Stuhlbein 27 aufweist und Mittel 29, 28 zum Vorgeben einer festgelegten Knieabwinklung über die Höhenverstellung vorgesehen sind, welche insbesondere eine Fußauflage-Kontaktplatte 29 umfassen, die zur Abgabe eines Schaltsignals HV_{AUS} zum Anhalten der Höhenverstellung des Stuhlbeins 27 bei aufsetzendem bzw. abhebendem Fuß eingerichtet ist.
- Punkt 5.: Messvorrichtung 1; 100 insbesondere nach Punkt 4, wobei die Mittel zum Vorgeben der festgelegten Knieabwinklung eine Starteinrichtung zum Start HV_{AN} der Höhenverstellung des Stuhlbeins 27 aufweist, die insbesondere bei aufgesetztem Fuß die Höhenverstellung nach oben auslöst und bei abgehobenem Fuß nach unten.
- Punkt 6.: Messvorrichtung 1; 100 nach Punkt 4 oder 5, wobei die Fußauflage-Kontaktplatte 29 mit einem Endschalter 28 verbunden ist, welcher bei aufsetzendem bzw. abhebendem Fuß einen Antrieb für die Höhenverstellung des Stuhlbeins 27 abschaltet HV_{AUS}.
- Punkt 7.: Messvorrichtung nach Punkt 4 oder 5, wobei die Fußauflage-Kontaktplatte mit einer Kontaktfolie versehen ist, welche bei aufsetzendem bzw. abhebendem Fuß den Antrieb für die Höhenverstellung des Stuhlbeins abschaltet.
- Punkt 8.: Messvorrichtung 1, wobei eine Sitzhöhen-Erfassungseinrichtung 17, 19 zum Erfassen der Sitzhöhe HV bei der festgelegten Knieabwinklung, insbesondere mit einer Lichtschranke 17 einer Inkrementalskala 19 vorgesehen ist.
- Punkt 9.: Messvorrichtung 1 nach Punkt 9, wobei die Recheneinrichtung 30 Mittel 32 zum Errechnen eines Beineinstellungsparameters UL des Fitnessgeräts aus der Sitzhöhe bei der festgelegten Knieabwinklung umfasst.
- Punkt 10.: Messvorrichtung 1 nach Punkt 8 oder 9, wobei die Recheneinrichtung 30 Mittel 31 zum Schätzen der Gesamtbeinlänge oder einer entsprechenden Einstellung BL des Fitnessgeräts aus der Sitzhöhe HV bei der festgelegten Knieabwinklung umfasst.
- Punkt 11.: Messvorrichtung 1, wobei die Recheneinrichtung 30 Mittel 31 zum Schätzen der Höhe des Hüftgelenkdrehpunkts in stehender Haltung der jeweiligen Person oder einer entsprechenden Einstellung des Fitnessgeräts aus der Sitzhöhe HV und/oder der Höhe des SchultergelenkDrehpunkts SH in der sitzenden Haltung bei der festgelegten Knieabwinklung umfasst.
- Punkt 12.: Messvorrichtung 1, wobei die Recheneinrichtung 30 Mittel 34 zum Errechnen einer Höhe des Schultergelenk-Drehpunkts SHA in einer stehenden Haltung oder einer entsprechenden Einstellung des Fitnessgeräts aus der Höhe des Schultergelenk-Drehpunkts SH in der sitzenden Haltung mit der festgelegten Knieabwinklung und der Gesamtbeinlänge BL umfasst.
- Punkt 13.: Messvorrichtung 1, wobei zumindest ein einer Körperbewegung AS der jeweiligen Person entgegenwirkend angebrachter Kraftaufnehmer 53, 55 vorgesehen ist, insbesondere eine Kraftmessdose 53, 55, eine Drehmomentmesswelle, oder eine Wirbelstrombremse, und die Recheneinrichtung 30 Mittel 35 zum Errechnen einer entsprechenden Einstellung UF des Fitnessgeräts aus der von dem Kraftaufnehmer 53, 55 erfassten Werten Fα, Fβ bei der von der jeweiligen Person aufzubringenden Krafteingabe F_{MAX} umfasst.
- Punkt 14.: Messvorrichtung 1 nach Punkt 13 mit einem ersten Kraftaufnehmer-Verdrehwiderstand 53, der zur Aufnahme einer auf die Anlenkung 13 des ersten Gelenkhebels 7 wirkenden Kraft Fα in einer vorgesehenen Stellung des Angriffsorgans zuschaltbar ist und einen zweiten Kraftaufnehmer-Verdrehwiderstand 55, der zur Aufnahme einer auf die Anlenkung 15 des zweiten Gelenkhebels 7 wirkenden Kraft Fβ in der vorgesehenen Stellung des Angriffsorgans zuschaltbar ist.
- Punkt 15.: Messvorrichtung 1 nach Punkt 13 oder 14 mit einem mit einer Brustpolsterrolle 23 verbundenen Kraftaufnehmer-Verdreh- oder Verschiebewiderstand.
- Punkt 16.: Messvorrichtung 1 nach Punkt 13, 14 oder 15 mit einem mit einer Beinpolsterrolle 24, 25 verbundenen Kraftaufnehmer-Verdreh- oder Verschiebewiderstand.
- Punkt 17.: Messvorrichtung 1 mit einem mit der Anlenkung 21 der Rückenlehne 5 an der Sitzfläche (3) verbundenen Kraftaufnehmer-Verdrehwiderstand 21.
- Punkt 18.: Messstuhl mit zumindest einer weiteren Körperparameter-Erfassungseinrichtung, insbesondere einer Waage, einem Beweglichkeitsmesser, einem Pulsmesser oder einem Körperfettanteilsmesser, zum Erzeugen zumindest eines Werts eines weiteren körperbezogenen Parameters der jeweiligen Person, wobei die Recheneinrichtung Mittel zur Errechnung einer ent- sprechenden Einstellung des Fitnessgeräts aufweist.
- Punkt 19.: Messvorrichtung 1, wobei die Recheneinrichtung Mittel 37 zur Errechnung eines Trainingsprogramms TP aus den erfassten Werten αAS_{AN}, αAS_{AUS}, βAS_{AN}, βAS_{AUS}, HV, Fα, Fβ umfasst, welches die FitnessgeräteEinstellparameter umfasst, wobei zumindest einer der Fitnessgeräte-Einstellparameter UF über die Zeit gemäß eines vorgegebenen Trainingsablaufs variiert.
- Punkt 20.: Messvorrichtung 1, wobei die Recheneinrichtung eine Speichereinrichtung 39 zum Speichern des Werts des zumindest einen körpergeometrie-, -kraft- oder sonstigen körperbezogenen Parameters, des zumindest einen Fitnessgeräte-Einstellparameters AL, RL, SH, BL, UL, UF und/oder des Trainingsprogramms TP auf einer Speicherkarte 41 aufweist.

## Patentansprüche

1. Messvorrichtung (1; 100) für eine Ersteinstellung von zumindest einem Fitnessgerät auf die Höhe und/oder Breitenlage eines Drehpunkts (SH) eines Gelenks von unterschiedlichen Personen, wie beispielsweise die Höhe des Schultergelenk-Drehpunkts (SH) in einer vorgegebenen Haltung der jeweiligen Person,
**dadurch gekennzeichnet, dass**
die Messvorrichtung (1; 100)
zum automatisierten Ermitteln von Werten (αAS_{AN}, αAS_{AUS}, βAS_{AN}, βAS_{AUS}) eingerichtet ist, die einer Höhe und/oder Breitenlage zumindest eines Drehpunkts (SH) zumindest eines Gelenks einer Person in einer vorgegebenen Haltung der Person entsprechen, aus zumindest einer standardisierten Körperbewegung (AS) der jeweiligen Person und
zur Bereitstellung der Werte (αAS_{AN}, αAS_{AUS}, βAS_{AN}, βAS_{AUS}) in maschinell weiterverarbeitbarer Form, wobei die standardisierte Körperbewegung (AS) eine Schwenkbewegung eines Körperteils (A) um das Gelenk um einen vorgegebenen Winkel ist.

2. Messvorrichtung (1; 100), nach Anspruch 1, mit:
einer Personenaufnahme (4, 5), welche insbesondere von einem Sitz (4, 5) mit einer Sitzfläche (4) und einer Rückenlehne (5) gebildet wird, zum Vorgeben der vorgegebenen Haltung der jeweiligen Person, insbesondere einer sitzenden Haltung,
zumindest einem Vorgabemittel (7, 9, 11; 107, 109, 111) zum Vorgeben zumindest eines erfassbaren Parameters (α, β), welcher eine Position des mit der vorgegebenen Körperbewegung (AS) um das Gelenk bewegten Körperteils (H) bei der Ausführung der vorgegebenen Körperbewegung (AS) wiedergibt, und zumindest einer Erfassungseinrichtung (13, 15; 113, 115) zum Erfassen von zumindest zwei Werten (αAS_{AN}, αAS_{AUS}, βAS_{AN}, βAS_{AUS}) des zumindest einen der Körperbewegung (AS) entsprechenden Parameters (α, β).

3. Messvorrichtung (1; 100) nach Anspruch 1 oder 2, mit
zumindest einer Recheneinrichtung (30), welche Mittel (33) zum Berechnen der Höhe des Gelenkdrehpunkts (SH) aus den Parameterwerten (αAS_{AN}, αAS_{AUS}, βAS_{AN}, βAS_{AUS}) umfasst.

4. Messvorrichtung (1; 100) nach Anspruch 1, 2 oder 3, **dadurch gekennzeichnet, dass** das Vorgabemittel (7, 9, 11; 107, 109, 111) zumindest ein gegenüber der Personenaufnahme (4, 5) mit Bewegungsfreiheitsgrad in Richtung der Körperbewegung (AS) angeordnetes, mit dem Körperteil (A) angreifbares Angriffsorgan (11; 111) zum geführten Ausführen der vorgegebenen Körperbewegung (AS) durch die jeweilige Person umfasst, welches gegenüber der Personenaufnahme (4, 5) eine solche Bewegungsfreiheit aufweist, dass Testpersonen unterschiedlicher Körpergeometrie die standardisierte Körperbewegung ausführen können.

5. Messvorrichtung nach einem der vorhergehenden Ansprüche, **dadurch gekennzeichnet, dass** das Vorgabemittel zumindest ein mit dem Körperteil verbindbares Trägerorgan umfasst und die Erfassungseinrichtung zumindest einen auf dem Trägerorgan aufgenommenen, an dem Körperteil ortsfest positionierbaren Positionssensor.

6. Messvorrichtung (1; 100) nach Anspruch 4 oder 5, **dadurch gekennzeichnet, dass** das Trägerorgan oder das Angriffsorgan (11; 111) zum Ausführen einer Armschwenkung (AS) mit zumindest einem, vorzugsweise beiden Armen (A) eingerichtet ist, und die Körperbewegung (AS) eine Armschwenkung (AS) um einen vorgegebenen Winkel, insbesondere 180° ist.

7. Messvorrichtung (1; 100) nach Anspruch 6, **dadurch gekennzeichnet, dass** das Träger- oder Angriffsorgan (11; 111) zumindest einen, vorzugsweise zwei Griffe (11; 111) umfasst, und die Körperbewegung (AS) eine Armschwenkung (AS) mit gestrecktem Arm bzw. gestreckten Armen um den vorgegebenen Winkel, insbesondere 180° ist.

8. Messvorrichtung (1; 100) nach Anspruch 7, **dadurch gekennzeichnet, dass** die Recheneinrichtung (30) Mittel (33) zum Berechnen der Armlänge (AL) aus den bei der mit gestrecktem Arm bzw. gestreckten Armen um den vorgegebenen Winkel ausgeführten Armschwenkung (AS) erfassten Parameterwerten (αAS_{AN}, αAS_{AUS}, βAS_{AN}, βAS_{AUS}) umfasst.

9. Messvorrichtung nach der Ansprüche 2 bis 8, **dadurch gekennzeichnet, dass** das Träger- oder Angriffsorgan zumindest eine, vorzugsweise zwei Fußschlaufen umfasst, und die Körperbewegung eine Beinschwenkung um einen bestimmten Winkel um das Kniegelenk ist.

10. Messvorrichtung nach einem der Ansprüche 2 bis 9, **dadurch gekennzeichnet, dass**
das Angriffsorgan einen Brustgurt umfasst, und die Körperbewegung eine Rumpfschwenkung um einen bestimmten Winkel um das Hüftgelenk ist.

11. Messvorrichtung (1; 100) nach einem der vorhergehenden Ansprüche, **dadurch gekennzeichnet, dass** das Vorgabemittel (7, 9, 11; 107, 109, 111) zumindest einen ersten Gelenkhebel (7; 107) umfasst, der um eine erste horizontale Achse (I) gegenüber der Personenaufnahme (4, 5) schwenkbar angelenkt ist, insbesondere an einem Rahmengestell des Sitzes (4, 5), beispielsweise im Bereich der Rückenlehne (5) oder an einem mit der Rückenlehne (5) verbundenen Holm (141), wobei das Angriffsmittel (11; 111) an dem dem Anlenkpunkt des ersten Gelenkhebels (7; 107) entgegengesetzten Ende des Vorgabemittels (7, 9, 11; 107, 109, 111) vorgesehen ist, und wobei das Angriffsmittel (11; 111) gegenüber der Personenaufnahme (4, 5) zumindest einen weiteren Bewegungsfreiheitsgrad (β) aufweist.

12. Messvorrichtung (1; 100) nach Anspruch 11, **dadurch gekennzeichnet, dass** die Erfassungseinrichtung (13, 15; 113, 115) zumindest eine erste Winkelerfassungeinrichtung (13; 113) zum Erfassen eines ersten Winkels (α) aufweist, welcher der bei der Körperbewegung (AS) erzeugten Schwenkbewegung des ersten Gelenkhebels (7; 107) um die erste horizontale Achse (I) entspricht, insbesondere ein Potentiometer (113) oder einen Inkrementalgeber (13).

13. Messvorrichtung (100) nach einem der vorhergehenden Ansprüche, **dadurch gekennzeichnet, dass** das Vorgabemittel (107; 109, 111) zumindest eine Linearführung (107) aufweist, insbesondere eine im wesentlichen vertikal entlang der Rückenlehne verlaufende Linearführung oder ein den ersten Gelenkhebel (107) bildendes Führungsrohr (107), in der ein mit dem Angriffsmittel (111) verbundener Schlitten (109) verfahrbar ist, wobei das Angriffsmittel (111) gegenüber der Personenaufnahme zumindest einen weiteren Bewegungsfreiheitsgrad (α) aufweist.

14. Messvorrichtung (100) nach Anspruch 13, **dadurch gekennzeichnet, dass** die Erfassungseinrichtung (113; 115) zumindest eine erste Längenerfassungseinrichtung (115) zum Erfassen eines Wegs (W) aufweist, der einer bei der Körperbewegung erzeugten Linearbewegung des Schlittens (109) entspricht, insbesondere einen Positionssensor (115) oder einen Inkrementalgeber.

15. Messvorrichtung (100) nach einem der Ansprüche 11 bis 14, **dadurch gekennzeichnet, dass** das Vorgabemittel (107, 109, 111) mit einem mitschwenkenden Dorn (144) verbunden ist, für den eine Anschlagsanordnung (142, 143) vorgesehen ist, so dass die Schwenkbewegung des ersten Gelenkhebels (107) auf einen festgelegten Winkelbereich begrenzt ist, insbesondere auf den vorgegebenen Winkel.

16. Messvorrichtung (1) nach einem der Ansprüche 11 bis 15, **dadurch gekennzeichnet, dass** das Vorgabemittel (7, 9, 11) zumindest einen zweiten Gelenkhebel (9) aufweist, der um eine zweite horizontale Achse (11) schwenkbar an dem ersten Gelenkhebel (7) angelenkt ist, und zwar an dem dem Anlenkpunkt an der Personenaufnahme (4, 5) entgegengesetzten Ende des ersten Gelenkhebels (7).

17. Messvorrichtung (1) nach Anspruch 16, **dadurch gekennzeichnet, dass** die Erfassungseinrichtung (13, 15) eine zweite Winkelerfassungseinrichtung (15) zum Erfassen eines zweiten Winkels (β) aufweist, welcher der bei der Körperbewegung erzeugten Schwenkbewegung des zweiten Gelenkhebels (9) um die zweite horizontale Achse (II) entspricht, insbesondere ein Potentiometer oder einen Inkrementalgeber (15).

18. Messvorrichtung (1; 100) nach einem der Ansprüche 11 bis 17, **dadurch gekennzeichnet, dass** das Vorgabemittel (7, 9, 11; 107, 109, 111) zumindest zwei symmetrisch zueinander aufgebaute Abschnitte (7, 9, 11; 107, 109, 111) zum beidseitigen Betätigung mit jeweils den beiden gleichen Körpergliedmaßen (A) aufweist, wobei die Abschnitte (7, 9, 11; 107, 109, 111) vorzugsweise über eine Synchronisierung, insbesondere über eine Synchronisienrwelle (149) miteinander verbunden sind.

## Claims

1. Measuring device (1; 100) for initially adjusting at least one exercise machine to the height and/or width position of a pivot point (SH) of a joint of different persons, such as the height of the shoulder-joint pivot point (SH) in a specified position of the respective person, **characterized in that** the measuring device (1; 100) is set up to automatically determine values (αAS_{AN}, αAS_{AUS}, βAS_{AN}, ßAS_{AUS}) - corresponding to a height and/or width position of at least one pivot point (SH) of at least one joint of a person in a specified position of the person - from at least one standardized body movement (AS) of the respective person and to make the values (αAS_{AN}, αAS_{AUS}, βAS_{AN}, βAS_{AUS}) available in machine-processable form, wherein the standardized body movement (AS) is a swivelling movement of a body part (A) about the joint through a specified angle.

2. Measuring device (1; 100) according to claim 1, with:
a seating arrangement (4, 5), said seating arrangement (4, 5) being formed more particularly by a seat (4, 5) with a seating surface (4) and a backrest (5), for specifying the specified position of the respective person, more particularly a seated position,
at least one specifying means (7, 9, 11; 107, 109, 111) for specifying at least one measurable parameter (α, β), said parameter (α, β) mirroring a position of the body part (H) moved about the joint by the specified body movement (AS) during execution of the specified body motion (AS), and at least one measuring arrangement (13, 15; 113, 115) for measuring at least two values (αAS_{AN}, αAS_{AUS} βAS_{AN}, βAS_{AUS}) of the at least one parameter (α, β) corresponding to the body movement (AS).

3. Measuring device (1; 100) according to claim 1 or 2, with
at least one computing arrangement (30), said computing arrangement (30) comprising means (33) for calculating the height of the pivot point (SH) of the joint from the parameter values (αAS_{AN}, αAS_{AUS}, ßAS_{AN}, ßAS_{AUS}).

4. Measuring device (1; 100) according to claim 1, 2 or 3, **characterized in that** the specifying means (7, 9, 11; 107, 109, 111) comprises at least one engagement member (11; 111), provided with a degree of freedom of movement in the direction of the body movement (AS) with respect to the seating arrangement (4, 5) and being engageable with the body part (A), for the controlled execution of the specified body movement (AS) by the respective person, said engagement member (11; 111) having such freedom of movement with respect to the seating arrangement (4, 5) that test persons of different body geometries are able to execute the standardized body movement.

5. Measuring device according to any one of the preceding claims, **characterized in that** the specifying means comprises at least one carrier member, connectable to the body part, and the measuring arrangement comprises at least one position sensor accommodated on the carrier member and fixedly positionable on the body part.

6. Measuring device (1; 100) according to claim 4 or 5, **characterized in that** the carrier member or the engagement member (11; 111) is set up for execution of an arm swing (AS) with at least one and preferably both arms (A), and the body movement (AS) is an arm swing (AS) through a specified angle, more particularly 180°.

7. Measuring device (1; 100) according to claim 6, **characterized in that** the carrier or engagement member (11; 111) comprises at least one and preferably two grips (11; 111), and the body movement (AS) is an arm swing (AS) with stretched arm(s) through the specified angle, more particularly 180°.

8. Measuring device (1; 100) according to claim 7, **characterized in that** the computing arrangement (30) comprises means (33) for calculating the arm length (AL) from the parameter values (αAS_{AN}, αAS_{AUS}, ßAS_{AN}, ßAS_{AUS}) measured during the arm swing (AS) executed through the specified angle with stretched arm(s).

9. Measuring device according to claims 2 to 8, **characterized in that** the carrier or engagement member comprises at least one and preferably two foot loops, and the body movement is a leg swing through a defined angle about the knee joint.

10. Measuring device according to any one of claims 2 to 9, **characterized in that** the engagement member comprises a chest strap, and the body movement is a torso swing through a defined angle about the hip joint.

11. Measuring device (1; 100) according to any one of the preceding claims, **characterized in that** the specifying means (7, 9, 11; 107, 109, 111) comprises at least one first articulated lever (7; 107), said first articulated lever (7; 107) being swivel-mounted with respect to the seating arrangement (4, 5) about a first horizontal axis (I), more particularly being swivel-mounted to a frame of the seat (4, 5), for example in the region of the backrest (5) or to a bar (141) connected to the backrest (5), wherein the engagement means (11; 111) is provided at the end of the specifying means (7, 9, 11; 107, 109, 111) opposite to the swivel-mounting point of the first articulated lever (7; 107), and wherein the engagement means (11; 111) has at least one additional degree of freedom of movement (β) with respect to the seating arrangement (4, 5).

12. Measuring device (1; 100) according to claim 11, **characterized in that** the measuring arrangement (13, 15; 113, 115) has at least one first angle-measuring arrangement (13; 113) for measuring a first angle (α) corresponding to the swivelling movement, generated during the body movement (AS), of the first articulated lever (7; 107) about the first horizontal axis (I), more particularly a potentiometer (113) or an incremental position encoder (13).

13. Measuring device (100) according to any one of the preceding claims, **characterized in that** the specifying means (107; 109, 111) has at least one linear guide (107), more particularly a linear guide extending substantially vertically along the backrest, or a guide tube (107) forming the first articulated lever (107), wherein a sled (109), connected to the engagement means (111), is movable in said linear guide (107), the engagement means (111) having at least one additional degree of freedom of movement (α) with respect to the seating arrangement.

14. Measuring device (100) according to claim 13, **characterized in that** the measuring arrangement (113; 115) has at least one first length-measuring arrangement (115) for measuring a distance (W) corresponding to a linear movement of the sled (109) generated during the body movement, more particularly a position sensor (115) or an incremental position encoder.

15. Measuring device (100) according to any one of claims 11 to 14, **characterized in that** the specifying means (107, 109, 111) is connected to a co-swivelling bolt (144), wherein an end-stop arrangement (142, 143) is provided for said bolt (144), with the result that the swivelling movement of the first articulated lever (107) is limited to a fixed angular range, more particularly to the specified angle.

16. Measuring device (1) according to any one or more of claims 11 to 15, **characterized in that** the specifying means (7, 9, 11) has at least one second articulated lever (9), said second articulated lever (9) being swivel-mounted to the first articulated lever (7) about a second horizontal axis (II), more specifically being swivel-mounted at the end of the first articulated lever (7) opposite the swivel-mounting point on the seating arrangement (4, 5).

17. Measuring device (1) according to claim 16, **characterized in that** the measuring arrangement (13, 15) has a second angle-measuring arrangement (15) for measuring a second angle (β) corresponding to the swivelling movement, generated during the body movement, of the second articulated lever (9) about the second horizontal axis (II), more particularly a potentiometer or an incremental position encoder (15).

18. Measuring device (1; 100) according to any one of claims 11 to 17, **characterized in that** the specifying means (7, 9, 11; 107, 109, 111) has at least two symmetrically disposed portions (7, 9, 11; 107, 109, 111) for two-sided operation with in each case the two identical limb dimensions (A), wherein the portions (7, 9, 11; 107, 109, 111) are interconnected preferably through the intermediary of a synchronizing means, more particularly through the intermediary of a synchronizing shaft (149).

## Revendications

1. Dispositif de mesure (1 ; 100) pour un réglage initial d'au moins un appareil de culture physique à la hauteur et/ou la position en largeur d'un point de rotation (SH) d'une articulation de différentes personnes, comme par exemple la hauteur du point de rotation (SH) d'une articulation d'épaule dans une posture spécifiée de la personne respective,
**caractérisé en ce que**
le dispositif de mesure (1 ; 100) est conçu
pour la détermination automatisée de valeurs (αAS_{AN}, αAS_{AUS}, βAS_{AN}, βAS_{AUS}), qui correspondent à une hauteur et/ou une position en largeur d'au moins un point de rotation (SH) d'au moins une articulation d'une personne, dans une position spécifiée de la personne à partir d'au moins un mouvement corporel (AS) normalisé de la personne respective et
pour la fourniture des valeurs (αAS_{AN}, αAS_{AUS}, βAS_{AN}, βAS_{AUS}) sous une forme pouvant être transformée ultérieurement de manière mécanique, le mouvement corporel (AS) normalisé étant un mouvement de pivotement d'une partie de corps (A) d'un angle spécifié autour de l'articulation.

2. Dispositif de mesure (1 ; 100), selon la revendication 1, comprenant :
Un dispositif d'accueil de personnes (4, 5), qui est formé en particulier d'un siège (4, 5) comprenant une surface d'assise (4) et un dossier (5), pour la spécification de la position spécifiée de la personne respective, en particulier une position assise,
au moins un moyen de spécification (7, 9, 11 ; 107, 109, 111) pour la spécification d'au moins un paramètre (α, β) enregistrable, qui reproduit une position de la partie de corps (H) déplacée autour de l'articulation par le mouvement corporel (AS) spécifié lors de l'exécution du mouvement corporel (AS) spécifié, et au moins un dispositif d'enregistrement (13, 15 ; 113, 115) pour l'enregistrement d'au moins deux valeurs (αAS_{AN}, αAS_{AUS}. βAS_{AN}, βAS_{AUS}) du au moins un paramètre (α, β) correspondant au mouvement corporel (AS).

3. Dispositif de mesure (1 ; 100) selon la revendication 1 ou 2, avec :
au moins un dispositif de calcul (30), qui comprend un moyen (33) pour calculer la hauteur du point de rotation (SH) de l'articulation à partir des valeurs de paramètre (αAS_{AN}, αAS_{AUS}, βAS_{AN}, βAS_{AUS}).

4. Dispositif de mesure (1 ; 100) selon la revendication 1, 2 ou 3, **caractérisé en ce que** le moyen de spécification (7, 9, 11 ; 107, 109, 111) est constitué d'au moins un élément de préhension (11 ; 111) pouvant être saisi par la partie de corps (A), disposé par rapport au dispositif d'accueil de personnes (4, 5), avec un degré de liberté de mouvement en direction du mouvement corporel (AS) pour la réalisation guidée du mouvement corporel spécifié (AS) par la personne respective, qui présente par rapport au dispositif d'accueil de personnes (4, 5) une liberté de mouvement telle que des sujets de géométrie corporelles différentes puissent réaliser le mouvement corporel normalisé.

5. Dispositif de mesure selon l'une quelconque des revendications précédentes, **caractérisé en ce que** le moyen de spécification comprend au moins un élément porteur pouvant être relié avec la partie de corps et le dispositif d'enregistrement comprend au moins un capteur de position accueilli sur l'élément porteur et pouvant être positionné de manière fixe au niveau de la partie de corps.

6. Dispositif de mesure (1 ; 100) selon la revendication 4 ou 5, **caractérisé en ce que** l'élément porteur ou l'élément de préhension (11 ; 111) est conçu pour la réalisation d'un pivotement de bras (AS) avec au moins un, de préférence deux bras (A), et le mouvement corporel (AS) est un pivotement de bras (AS) d'un angle spécifié, en particulier 180°.

7. Dispositif de mesure (1 ; 100) selon la revendication 6, **caractérisé en ce que** l'élément porteur ou de préhension (11 ; 111) comprend au moins une, de préférence deux poignées (11 ; 111), et le mouvement corporel (AS) est un pivotement de bras (AS) de l'angle spécifié, en particulier 180°, avec un bras allongé ou les bras allongés.

8. Dispositif de mesure (1 ; 100) selon la revendication 7, **caractérisé en ce que** le dispositif de calcul (30) comprend un moyen (33) pour calculer la longueur de bras (AL) à partir des valeurs de paramètre (αAS_{AN}, αAS_{AUS}, βAS_{AN}, βAS_{AUS}) enregistrées par le pivotement de bras (AS) de l'angle spécifié réalisé par le bras allongé ou les bras allongés.

9. Dispositif de mesure selon les revendications 2 à 8, **caractérisé en ce que** l'élément porteur ou de préhension comprend au moins une, de préférence deux boucles pour les pieds, et le mouvement corporel est un pivotement de jambe d'un angle déterminé autour de l'articulation du genou.

10. Dispositif de mesure selon l'une quelconque des revendications 2 à 9, **caractérisé en ce que** l'élément de préhension comprend une sangle pectorale, et le mouvement corporel est un pivotement du tronc d'un angle déterminé autour de l'articulation de la hanche.

11. Dispositif de mesure (1 ; 100) selon l'une quelconque de revendications précédentes, **caractérisé en ce que** le moyen de spécification (7, 9, 11 ; 107, 109, 111) comprend au moins un premier levier articulé (7 ; 107), qui est articulé de manière pivotante autour d'un premier axe horizontal (I) par rapport au dispositif d'accueil de personnes (4, 5), en particulier au niveau d'un châssis du siège (4, 5), par exemple dans le secteur du dossier (5) ou au niveau d'un montant (141) relié au dossier (5), le moyen de préhension (11 ; 111) étant prévu au niveau de l'extrémité opposée au point d'articulation du premier levier articulé (7, 107) du moyen de spécification (7, 9, 11 ; 107, 109, 111), et le moyen de préhension (11 ; 111) présentant au moins un autre degré de liberté de mouvement (β) par rapport au dispositif d'accueil de personnes (4, 5).

12. Dispositif de mesure (1 ; 100) selon la revendication 11, **caractérisé en ce que** le dispositif d'enregistrement (13, 15 ; 113, 115) présente au moins un premier dispositif d'enregistrement d'angle (13 ; 113) pour l'enregistrement d'un premier angle (α), qui correspond au mouvement de pivotement, produit par le mouvement corporel (AS), du premier levier articulé (7 ; 107) autour du premier axe horizontal (10), en particulier un potentiomètre (113) ou un transmetteur incrémental (13).

13. Dispositif de mesure (100) selon l'une quelconque des revendications précédentes, **caractérisé en ce que** le moyen de spécification (107 ; 109, 111) présente au moins un guidage linéaire (107), en particulier un guidage linéaire passant essentiellement de manière verticale le long du dossier ou un tube de guidage (107) formant le premier levier articulé (107), dans lequel un chariot (109) relié avec le moyen de préhension (111) peut être déplacé, le moyen de préhension (111) présentant au moins un autre degré de liberté de mouvement (α) par rapport au dispositif d'accueil de personnes.

14. Dispositif de mesure (100) selon la revendication 13, **caractérisé en ce que** le dispositif d'enregistrement (113 ; 115) présente au moins un premier dispositif d'enregistrement de longueur (115) pour l'enregistrement d'un chemin (W), qui correspond à un déplacement linéaire du chariot (109) produit par le mouvement corporel, en particulier un capteur de position (115) ou un transmetteur incrémental.

15. Dispositif de mesure (100) selon l'une quelconque des revendications 11 à 14, **caractérisé en ce que** le moyen de spécification (107, 109, 111) est relié avec un mandrin (144) co-pivotant, pour lequel un agencement de butée (142, 143) est prévu, de sorte que le mouvement de pivotement du premier levier articulé (107) est limité à une plage angulaire déterminée, en particulier à l'angle spécifié.

16. Dispositif de mesure (1) selon l'une des revendications 11 à 15, **caractérisé en ce que** le moyen de spécification (7, 9, 11) présente au moins un second levier articulé (9), qui est articulé de manière pivotante au niveau du premier levier articulé (7) autour d'un second axe horizontal (II), et de ce fait au niveau de l'extrémité, opposée au point d'articulation au niveau du dispositif d'accueil de personnes (4, 5), du premier levier articulé (7).

17. Dispositif de mesure (1) selon la revendication 16, **caractérisé en ce que** le dispositif d'enregistrement (13, 15) présente un second dispositif d'enregistrement d'angle (15) pour enregistrer un second angle (β), qui correspond au mouvement de pivotement, produit lors du mouvement corporel, du second levier articulé (9) autour du second axe horizontal (II), en particulier un potentiomètre ou un transmetteur incrémental (15).

18. Dispositif de mesure (1 ; 100) selon l'une quelconque des revendications 11 à 17, **caractérisé en ce que** le moyen de spécification (7, 9, 11 ; 107, 109, 111) présente au moins deux sections (7, 9, 11 ; 107, 109, 111) disposées symétriquement les unes par rapport aux autres pour un actionnement bilatéral avec respectivement les deux mêmes extrémités corporelles (A), les sections (7, 9, 11 ; 107, 109, 111) étant de préférence reliées les unes avec les autres par l'intermédiaire d'une synchronisation, en particulier par l'intermédiaire d'un arbre de synchronisation (149).
